(19) Europäisches Patentamt
European Patent Office
Office européen des brevets

(11) **EP 3 888 525 A1**

(12) **EUROPEAN PATENT APPLICATION**

(43) Date of publication:
**06.10.2021 Bulletin 2021/40**

(51) Int Cl.:
**A61B 3/028** *(2006.01)*     **A61B 3/09** *(2006.01)*

(21) Application number: **20382251.5**

(22) Date of filing: **31.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitat Politècnica De Catalunya**
**08034 Barcelona (ES)**

(72) Inventors:
• **GARCÍA GUERRA, Carlos Enrique**
**08222 Terrassa (ES)**

• **VILASECA RICART, Meritxell**
**08222 Terrassa (ES)**
• **DÍAZ DOUTÓN, Fernando**
**08222 Terrassa (ES)**
• **PUJOL RAMO, Jaume**
**08232 Viladecavalls (ES)**

(74) Representative: **Juncosa Miró, Jaime et al**
**Torner, Juncosa i Associats, S.L.**
**C/Pau Claris, 108, 1r 1a**
**08009 Barcelona (ES)**

(54) **METHOD AND COMPUTER PROGRAMS TO CHARACTERIZE EYE ACCOMMODATION**

(57)     A method and computer programs for characterizing ocular accommodation are provided. The method comprises measuring spherical refractive errors of an eye for different additional optical powers with respect to a reference refraction; calculating the behavior of an accommodation signal of the eye from the measured spherical refractive errors and from the additional optical powers; analyzing the behavior of the accommodation signal by calculating its differences with respect to the expected behavior in an eye without accommodative capacity by means of calculating the errors between the measured data and a fitting curve; and determining that there is a change in trend in the accommodation signal if a result of the analysis is greater than a threshold. When a change in trend occurs, the optical power at which the change has occurred is furthermore determined by means of the analysis of a curve of the accommodation signal.

Fig. 2

## Description

Technical Field

The present invention generally relates to objective methods for characterizing ocular accommodation in real time. Particularly, the invention relates to a method and to computer programs for determining the behavior of accommodation around a specific refraction.

Background of the Invention

Refraction is one of the most common tasks performed by optometrists and ophthalmologists clinical practice. This process seeks to quantify the refractive error of the patient (near-sightedness, far-sightedness, presbyopia, and astigmatism) for subsequent correction. This test often consists of presenting optotypes of different sizes and shapes to the patient while test lenses with different optical powers are placed in front of the eye. The purpose of the test is to determine the lens with the most positive optical power with which the best visual acuity is achieved (recognition of the smallest optotype) based on the subjective responses of the patient.

Subjective refraction, the name given to the process described above, fails when the patient involuntarily accommodates during the test as a consequence of the capacity of the eye to focus on objects at different distances as a result of changes in shape of the crystalline lens, namely due to its thickening. Involuntary accommodation primarily affects children and young adults since it is easier for them to accommodate. When this occurs during refraction, the patient may later express discomfort with his or her prescription, and the test has to be repeated.

There are several techniques for relaxing the accommodation during subjective refraction. For example, in the fogging technique, accommodation is relaxed by introducing a lens that is more positive than the expected spherical refraction (the eye is made to be nearsighted so that it will not accommodate); subsequently, the optical power is gradually reduced until finding the most positive test lens with which the patient achieves the best visual acuity. The success of this technique depends to a certain extent on the skill of the person performing the test to ensure non-accommodation of the eye for the refraction found. Another technique is to use diagnostic drugs (drops) to paralyze the muscle in charge of controlling accommodation (ciliary muscle). However, in addition to being invasive, this technique limits vision quality for a number of hours, so it may be uncomfortable for the patient.

Accommodation behavior can be estimated indirectly from the objective measurement of spherical refractive errors of the eye. For example, a Hartmann-Shack wavefront sensor capable of measuring in real time could be used to estimate both the microfluctuations in accommodation and its steady-state response. Said capacity of instruments of this type could be used during subjective refraction as a supporting tool which serves to objectively ensure that the steady-state accommodation response does not interfere with the result of the refraction test performed by the optometrist or ophthalmologist.

In clinical practice, the use of instruments capable of objectively measuring spherical and cylindrical errors of the eye is primarily used to obtain a prior estimate that serves as a starting point for conventional subjective refraction.

Objective-subjective refraction methods have been proposed in which the spherical and cylindrical refractive error of the eye is first obtained objectively; subsequently, the sphere is subjectively refined based on the initial target value (i.e., with a conventional subjective refraction method), whereas the cylindrical error objectively obtained is assumed to be true and not subjectively adjusted. Patents US10444539B2 and EP2369972B1 are examples of proposals of this type. One of the reasons for which in methods of this type the final value of the spherical refractive error is obtained subjectively is precisely for ensuring that the eye does not accommodate for the error found.

Patents US8985768B2 and US9462939B2 present systems which mimic the functionalities of a motorized phoropter (i.e., a clinical instrument normally used during refraction to place test lenses in front of the eye). The systems proposed in said patents feature a Hartmann-Shack wavefront sensor. The objective measurements provided by the sensor are used by the system itself to correct measured spherical and cylindrical errors and to make estimates of the refraction. Despite the capabilities of systems of this type and of their importance during subjective refraction, accommodation is not monitored.

Patent application US2011013140 presents a system based on a Hartmann-Shack wavefront sensor which can be coupled to a conventional phoropter. The system and methods proposed in this patent seek to make an estimate of the subjective refraction from the objective data provided by the wavefront sensor. For that purpose, spherical errors are monitored while additional optical powers placed in front of the eye with a phoropter are scanned, for example. For each additional power, the fluctuations of a vision quality indicator, for example, the standard deviation of the RMS (root mean square) error of the wavefront calculated from the Zernike coefficients describing it are quantified. The patent asserts that the magnitude of the fluctuations is related to patient comfort in the event of a visual stimulus. On this basis, the estimator of the subjective refraction obtained objectively will be the value of the induced lens for which there are fewer fluctuations in the selected vision quality indicator. The observed fluctuations would be related to the dynamic component of accommodation. However, the methods proposed in the patent do not monitor the component in steady-state.

Accommodation has been widely studied in scientific settings. A summary of the different characteristics studied can be consulted in references [1] and [2]. The studies performed up until now have generally analyzed the different components of accommodation independently and can be used to intuitively know their behavior. However, studies analyzing the component in accommodation steady-state during subjective refraction have not been found.

There is, therefore, a need for new methods which allow objectively characterizing the behavior of the component in accommodation steady-state. These methods could be applied during subjective refraction as a supporting tool for the optometrist or ophthalmologist for making better decisions during the test. Their use could also reduce uncertainty of the test as they depend to a lesser extent on the skills of the person in charge of the test to ensure the absence of accommodation in the eye for the subjective refraction found.

References:

[1] W. N. Charman and G. Heron, "Microfluctuations in accommodation: An update on their characteristics and possible role", Ophthalmic Physiol. Opt., vol. 35, no. 5, pp. 476-499, 2015, doi: 10.1111/opo.12234.

[2] W. N. Charman, "The eye in focus: accommodation and presbyopia", Clin. Exp. Optom., vol. 91, no. 3, pp. 207-225, 2008, doi: 10.1111/j.1444-0938.2008.00256.x.

Disclosure of the Invention

Embodiments of the present invention provide, according to a first aspect, a method for characterizing ocular accommodation, the method comprising measuring, based on information acquired about an eye of a user, the spherical refractive errors of said eye for different additional optical powers (defocusing) introduced by means of lenses or lens systems placed between the eye and the visual stimulus with respect to a reference refraction value; calculating, by means of a processor, the behavior of an accommodation signal of the eye from the measured spherical refractive errors and from the different additional optical powers used; and analyzing, by means of the processor, the behavior of the accommodation signal by quantifying the differences with respect to the expected behavior in an eye without accommodative capacity by means of calculating the errors between the accommodation signal and a fitting curve, and determining that there is a change in trend in the accommodation signal of the eye if the result of said analysis is greater than a specific threshold value.

According to the invention, when the preceding result indicates that a change in trend in the behavior of accommodation of the eye has occurred, the method further comprises determining the optical power at which the change in trend has occurred based on the analysis of a curve of the accommodation signal. The mentioned analysis comprises performing polynomial fits for each of the additional optical power values used.

The optical powers used can refer to the optical powers of a phoropter, of test glasses, or of lenses or systems of variable optical power, among others.

In a particular embodiment, the information acquired about the eye comprises one or more images of the eye acquired with an optical wavefront sensor, such as a Hartmann-Shack sensor. In other embodiments, sensors based on technologies other than wavefront sensors could be used for the same purpose, provided that refractive errors could be measured while scanning for additional optical powers with respect to a reference refraction.

In an embodiment, the mentioned polynomial fits include two polynomial fits. Likewise, for a specific additional optical power the method comprises performing a first fit considering the points of the accommodation signal which meet the condition that the additional optical power $\Delta P_{phor}$ is greater than or equal to said specific additional optical power, with a first fitting curve being obtained as a result; and performing a second fit considering the points of the accommodation signal which meet the condition that the additional optical power $\Delta P_{phor}$ is less than or equal to said specific optical power, with a second fitting curve being obtained as a result.

The difference existing between the first and second fitting curves and the accommodation signal can also be quantified, for example, by means of an RMS (root mean square error), absolute error, or SME (square mean error) algorithm, among others.

In an embodiment, the total difference between the fitting curves and the accommodation signal is calculated for an additional power determined as the sum of the previously quantified differences between the first and second fitting curve and the accommodation signal. The specific additional optical power in which the change in trend occurred will be the one with the smallest total difference.

In an embodiment, the mentioned analysis of the behavior of the accommodation signal to know if there is a change in trend is performed by means of fitting the accommodation signal to a second degree polynomial such that $y=a_2.x^2+a_1.x+a_0$; if $a_1$ and $a_2$ are greater than said threshold value, it would indicate, respectively, that the patient accommodates and/or that there is a change in trend in the range of additional optical powers used. Alternatively, in another embodiment, the mentioned analysis is performed by means of calculating the slope of the accommodation signal; if the value of the slope exceeds said threshold value for an additional optical power, it indicates that there is a change in trend.

For example, a threshold value which can be used during the analysis of the slope of the accommodation signal is 0.125 (change in the accommodation signal of 0.25 D in a range of ±1 D) as it is a value associated with the resolution commonly used in phoropters and in clinical practice. In other embodiments, particularly in cases where a more or less restrictive classification is required, different threshold values could be used, for example, 0.25 or 0.083 (changes in the accommodation signal of 0.25 D in a range of 1 and 3 D, respectively).

In an embodiment, the additional optical powers are comprised in a range between ±1 to ± 2 D with respect to the reference refraction. At present, both the paths between one range and another and between the different additional powers in said ranges are limited to 0.25 D, as it is the conventional resolution in clinical practice. In any event, the paths could be smaller with a phoropter or another lens system that allows it.

In an embodiment, prior to calculating the behavior of the accommodation signal of the eye the method comprises performing non-valid data filtration and averaging of said information acquired about the eye.

Other embodiments of the invention disclosed herein also include computer programs products to perform the steps and operations of the method proposed in the first aspect of the invention. More particularly, a computer program product is an embodiment that has a computer-readable medium including code instructions encoded therein which, when executed in at least one processor of the computer system, cause the processor to perform the operations indicated herein as embodiments of the invention.

Brief Description of the Drawings

The preceding and other features and advantages will be more fully understood based on the following merely illustrative and non-limiting detailed description of embodiments in reference to the attached drawings, in which:

Fig. 1 shows by way of example a schematic view of a system that can be coupled to a phoropter to implement the proposed method, according to an embodiment of the present invention.

Fig. 2 is a flow chart illustrating an embodiment of the proposed method.

Fig. 3 shows an example of the behavior of the spherical refractive error of the eye (A) and of the accommodation (B) for different additional optical powers with respect to a reference value placed in front of the eye.

Detailed Description of the Invention and Embodiments

The present invention provides a method for estimating the behavior of accommodation of an eye of a patient around a given refraction, which may be the refraction obtained objectively with an autorefractor or another independent system, or subjectively with the aid of a phoropter or other lens systems. The proposed method analyzes the measured behavior so as to estimate, if there is one, the optical power for which accommodation of the eye within the measured range is activated.

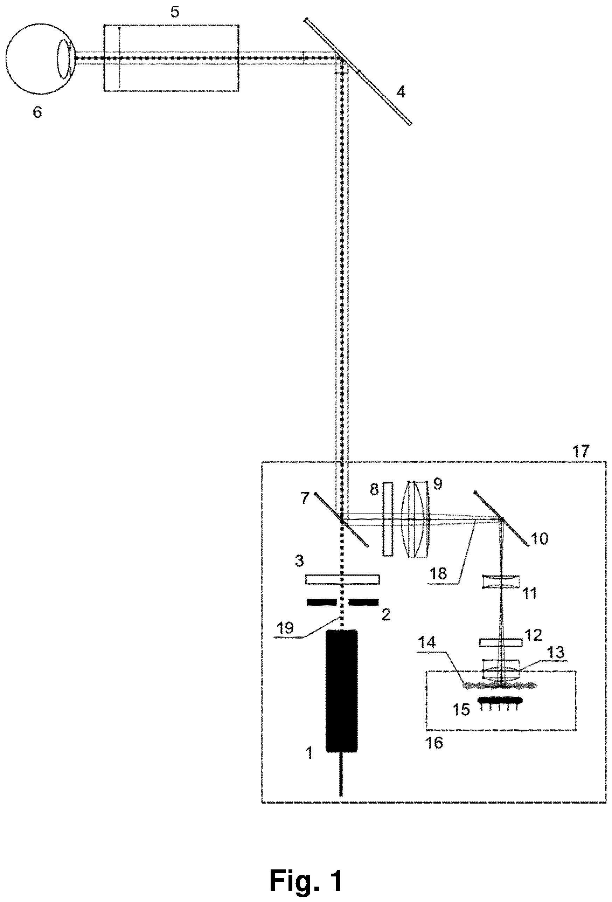

Fig. 1 shows an example of a system that can be used to implement the proposed method. According to this example, the system 17 is coupled to a phoropter 5 for measuring the spherical refraction changes of the eye 6 so as to estimate the behavior of the steady-state component of the accommodation. A hot mirror 4, which reflects infrared light while it transmits visible light, is used for coupling the system 17 to the phoropter 5 without interfering with the visual field of the patient. In the first path 19, a light source 1 emits a collimated beam, preferably in the near infrared, for example, at 780, 830, or 980 nm. The use of this part of the spectrum helps to prevent glares, resulting in greater comfort for the patient. The beam is limited with a diaphragm 2 at a diameter of between 1 mm and 2 mm such that it can be limited by diffraction upon passing through the lens of the eye 6. The light passes through the linear polarizer 3, is transmitted by the beam splitter 7, is reflected by the hot mirror 4, and passes through the lens of the phoropter 5 before reaching the eye 6.

In the second path 18, the light reflected by the retina inversely follows the route of the first path 19 described above until reaching the beam splitter 7. The light reflected by this element reaches the linear polarizer 8, the axis of polarization of which is oriented perpendicular to that of the linear polarizer 3 of the first path 19 for reducing corneal reflexes. Subsequently, the beam passes through the lens 9, is reflected by the mirror 10, and passes through the lens 11, the band-pass filter 12 and the lens 13 until reaching the wavefront sensor 16 (for example a Hartmann-Shack sensor) formed by the array of microlenses 14 followed by a CCD (charge coupled device) or CMOS (complementary metal oxide semiconductor) type image sensor 15 at a distance equal to the focal distance of the microlenses 14.

The positive lenses 9 and 13 form a telescope. Particularly, the example of Fig. 1 also includes a field lens 11, i.e., a lens with negative or positive optical power introduced in the optical route of the telescope in the focus of the lens 9 to modify (zoom out or in on) the image plane and ensure that the position of the eye is conjugate to the position of the array of microlenses 14 of the Hartmann-Shack sensor 16. This configuration allows having a compact system despite the increase in distance needed for coupling the system without interfering with the handling of the phoropter 5 during subjective refraction. For example, in a configuration with a field lens 11 with zero optical power (or without a field lens 11), with a telescope formed by lenses 9 and 13 with a focal distance of 50 mm and 30 mm, respectively, and a distance of about 2 mm between the lens 13 and the array of microlenses 14, the lens 9 should be placed 128 mm from the plane of measurement so as to have the eye 6 and the array of microlenses 14 in conjugate planes. This distance would be increased up to 267 mm upon introducing a field lens 11 of -18 D without modifying the rest of the configuration of the system. Other values of field lenses 11 could be used to configure the distance to the required value. That is, introducing the field lens 11 would allow having a system capable of modifying the plane of measurement (position of the eye) without modifying the rest of its configuration, ensuring that the plane of measurement and the array of microlenses 14 are always located in conjugate planes.

In an alternative configuration, the field lens 11 could be eliminated or be based on a lens of variable optical power for the dynamic configuration of the plane of measurement according to system usage needs.

In regard to the band-pass filter 12, said element has a transmission peak around the emission wavelengths of the laser for taking measurements unaffected by variations in lighting conditions.

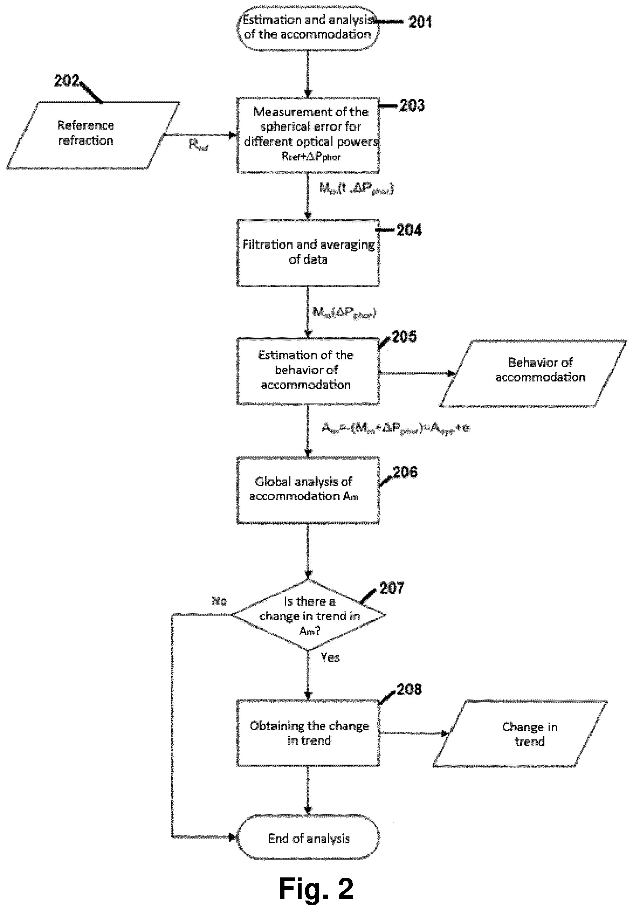

With reference to Fig. 2, therein it is showed an embodiment of the proposed method for characterizing ocular accommodation (step 201), particularly the steady-state behavior of the component, in relation to a reference refraction value (step 202). The method is executed, by one or more processors of a computing device/system, on information acquired about the eye 6 of a patient, for example one or more images of the eye 6 acquired, for example, by using the system of Fig. 1. Other systems based on other operating principles could be used to apply the proposed method, provided that said systems allow measuring refractive errors of the eye for different additional powers placed in front of the eye with respect to a reference refraction.

As described in step 203, first spherical refractive errors $M_m$ are measured for different additional optical powers $\Delta P_{phor}$, for example from $\pm 1$ to $\pm 2$ D (with additional lenses of spherical power of the phoropter 5) in steps of 0.25 D with respect to a reference refraction $R_{ref}$ to be corroborated.

For example, $R_{ref}$ could refer to the refraction of the patient obtained subjectively at a prior time or to an initial estimate of the spherical refractive error obtained by the system itself or with an external system. For each power $\Delta P_{phor}$ the number of measurements $M_m$ (between 10 and 100) needed to be able to estimate the steady-state behavior of accommodation are recorded, while the patient is asked to keep looking at a position on the eye chart. As an alternative to the phoropter 5, the additional optical powers could be induced with test lenses, with a lens system, or with lenses of variable optical power, such as electro-optical lenses or the like. In addition to the spherical refractive errors $M_m$, additional estimates which aid in processing the recorded data can be made during measurements. For example, data on grey levels in the information acquired about the eye 6 may be saved.

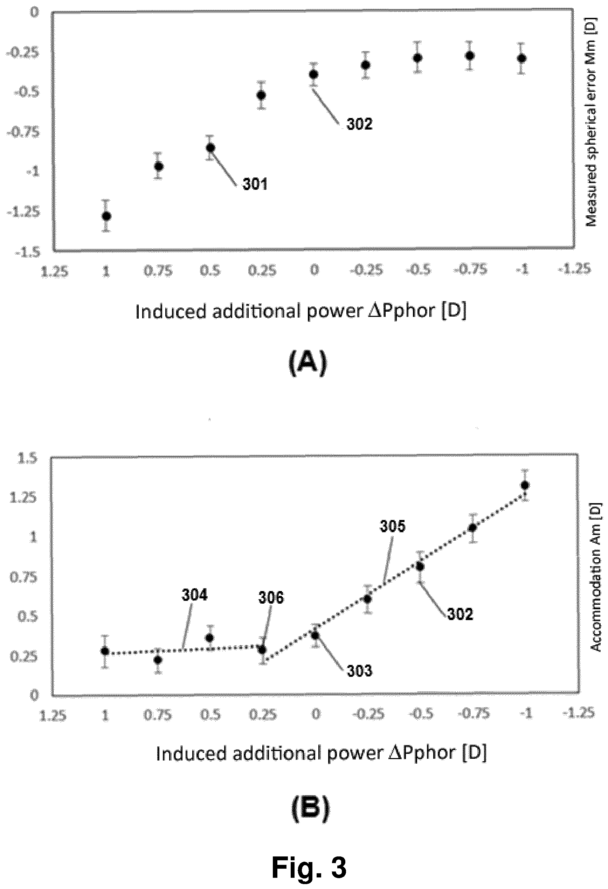

In step 204, the data recorded in the preceding point is first filtered to eliminate non-valid estimates. For example, statistical information about grey levels of the images (maximum values, minimum values, mean values, and standard deviations) could be used to detect sudden deviations that may be related to undesired events, such as the patient blinking. Once this data is filtered, the average 301 and its standard deviation 302 are obtained as estimators of the measured spherical refractive error $M_m$ for each of the additional induced optical powers $\Delta P_{phor}$. Fig. 3A shows an example of the data obtained after this process for measurements in an eye with accommodative capacity.

In other embodiments, in this case not illustrated, step 204 is not performed and the data recorded in step 203 is processed directly without performing filtration and averaging thereof.

The measured spherical errors $M_m$ may include the contribution of errors $M_{phor}$ and $M_{eye}$ introduced by the phoropter 5 and the eye 6, respectively:

$$M_m = M_{phor} + M_{eye}$$

These errors may be defined as follows:

$$M_{phor} = -\left(R_{ref} + \Delta P_{phor}\right)$$

$$M_{eye} = R_{eye} - A_{eye} = (R_{ref} - e) - A_{eye}$$

where $R_{eye}$ represents the refraction of the eye defined as the spherical refractive error when the accommodation $A_{eye}$ has a zero value (relaxed accommodation); error e represents the difference between the reference spherical refraction $R_{ref}$ and the refraction of the eye $R_{eye}$.

In step 206, the behavior of accommodation $A_m$ is estimated from the measured spherical errors $M_m$ and from the additional induced optical power $\Delta P_{phor}$, for example, by applying the following equation:

$$A_m = -\left(M_m + \Delta P_{phor}\right) = A_{eye} + e.$$

As e is a constant value, the behavior of $A_m$ follows the same way as described for the accommodation of the eye $A_{eye}$. However, the magnitude may not be representative, depending on the difference between the reference refraction $R_{ref}$ and the real refraction of the eye $R_{eye}$.

Fig. 3B shows an example of accommodation $A_m$ for an eye with accommodative capacity for different powers $\Delta P_{phor}$ around a reference refraction $R_{ref}$. It can be seen in the graph that the steady-state component of accommodation 303 has a virtually constant magnitude for $\Delta P_{phor}$ between +1 and +0.25 D. For lower optical powers, accommodation of the eye is activated, with the

magnitude of $A_m$ increasing. Said information could be provided to optometrists and ophthalmologists for visually corroborating the behavior of accommodation in relation to a refraction of interest, such as the refraction obtained objectively or in a first subjective estimate. The data depicted in Fig. 3B shows that accommodation is activated for powers starting from $P=R_{ref}+0.25$ D for this patient in particular.

In addition to said qualitative visual analysis by the person in charge of the test, the behavior of accommodation can be analyzed objectively as described in detail below. In process 206, accommodation $A_m$ is fit to a second degree polynomial such that $y=a_2x^2+a_1x+a_0$. Values of $a_1>0.125$ and $a_2>0.125$ would indicate, respectively, that the patient accommodates during the test and that there is a change in trend in the accommodation signal in the measured range. Alternatively, the existence of accommodation and the change in trend could be determined from an analysis of the slopes of the accommodation signal $A_m$. It may be concluded that the eye 6 accommodates if slopes $m>0.125$ are detected at any point of the curve. This analysis could also be used first to detect if there is more than one change in trend in the accommodation signal, and then to define the range of values $\Delta P_{phor}$ within which the transition between non-accommodation and accommodation of the eye 6 is observed for subsequent analysis. Polynomials of different degrees or fitting curves of another type could be used for the same purpose. Other limits/thresholds could also be chosen based on experience of the user.

When the process 206 indicates that there is a transition in the accommodation signal, the curve $A_m$ will be analyzed to find the induced optical power $\Delta P_{phor}$ at which said transition 306 occurs. For that purpose, polynomial fits could be performed for each of the values of induced powers $\Delta P_{phor}$ within the analyzed range.

In an embodiment, for a given power $\Delta P_{phor\_i}$, the first fit considers all the points of the accommodation signal $A_m$ which meet the condition $\Delta P_{phor}\geq\Delta P_{phor\_i}$. Similarly, the second fit considers the accommodation values for $\Delta P_{phor}\leq\Delta P_{phor\_i}$. Two fitting curves $Y_1$ and $Y_2$, with their respective error signals $E_{\gamma1}$ and $E_{\gamma2}$, are thereby obtained for each of the analyzed powers $\Delta P_{phor}$. Any estimator of the differences existing between fitting curves $Y_1$ and $Y_2$ and the accommodation signal, such as RMS error or SME, for example, could be used as error signals $E_{\gamma1}$ and $E_{\gamma2}$. The power $\Delta P_{phor}$ at which the transition occurs would be the one for which the pair $Y_1$-$Y_2$ has a minimum total error value $E_T=E_{Y1}+E_{Y2}$. Fig. 3B includes the selected fitting curves 304 and 305 obtained following this method. Said fitting curves describe the non-accommodation and accommodation region, respectively, after the transition 306.

In addition to the behavior of accommodation $A_m$, the proposed method allows providing the additional optical power $\Delta P_{phor\_ct}$ with respect to the reference spherical refraction $R_{ref}$ for which the change in transition 306 is observed. This information could be used to obtain the refraction $R_{ct}=R_{ref}+\Delta P_{phor\_ct}$ which would be defined as the optical power for which transition 306 occurs. Ideally $R_{ct}$ should coincide with the refraction of the eye $R_{eye}$. In cases where the spherical refraction obtained subjectively is different from $R_{ct}$, the optometrist or ophthalmologist in charge of the test should corroborate the result of the subjective test before deciding on the best prescription for the patient.

The method can be applied directly on the measured spherical refractive errors $M_m$. However, the accommodation signal $A_m$ is preferably used because its behavior is what turns out to be of interest during subjective refraction.

The proposed invention can be implemented in hardware, software, firmware, or any combination thereof. If implemented in software, the functions can be stored or encoded as one or more instructions or code in a computer-readable medium.

The scope of the present invention is defined in the attached claims.

## Claims

1. A method for characterizing ocular accommodation, the method comprising:

   a) measuring, based on information acquired about an eye of a user, spherical refractive errors of said eye for different additional optical powers introduced by means of lenses or lens systems placed between the eye and the visual stimulus with respect to a reference refraction value;

   b) calculating, by a processor, the behavior of an accommodation signal of the eye from the measured spherical refractive errors and from the different additional optical powers used;

   c) analyzing, by the processor, the behavior of the accommodation signal by calculating its differences with respect to the expected behavior in an eye without accommodative capacity by calculating the errors between the accommodation signal and a fitting curve and determining that there is a change in trend in the accommodation signal of the eye if a result of said analysis is greater than a specific threshold value; wherein said result indicates that a change in trend in the behavior of accommodation of the eye has occurred, the method further comprising:

   d) determining the optical power at which the change in trend has occurred based on the analysis of a curve of the accommodation signal, wherein said analysis comprises performing polynomial fits for each of the additional optical power values used.

**2.** The method according to claim 1, wherein said polynomial fits comprise two polynomial fits, and wherein for a specific additional optical power the method comprises:

- performing a first fit considering the points of the accommodation signal which meet the condition that the additional optical power $\Delta P_{phor}$ is greater than or equal to said specific additional optical power, with a first fitting curve being obtained as a result; and
- performing a second fit considering the points of the accommodation signal which meet the condition that the additional optical power $\Delta P_{phor}$ is less than or equal to said specific optical power, with a second fitting curve being obtained as a result.

**3.** The method according to claim 2, further comprising calculating the difference between the first and second fitting curves and the accommodation signal.

**4.** The method according to claim 3, wherein said difference is calculated using an RMS algorithm, absolute error, or SME.

**5.** The method according to claim 1, wherein said analysis of step c) comprises the calculation of a second degree polynomial or a different degree such that $y = a_2 \cdot x^2 + a_1 \cdot x + a_0$, wherein if its coefficients $a_1$ and $a_2$ are greater than said threshold value, it indicates that the eye accommodated and/or that there is a change in trend in the accommodation signal in the range of additional optical powers used.

**6.** The method according to claim 1, wherein said analysis of step c) comprises the calculation of the slope of the accommodation signal, wherein if the value of the slope is greater than said threshold value for any additional optical power, it indicates that there is a change in trend in the measured range.

**7.** The method according to claim 1, 5, or 6, wherein the threshold value is comprised in a range between 0.083 - 0.25 D.

**8.** The method according to claim 1, 5, 6, or 7, wherein the threshold value is 0.125 D.

**9.** The method according to any one of the preceding claims, wherein the additional optical powers are comprised in a range between $\pm 1$ to $\pm 2$ D with respect to the reference refraction.

**10.** The method according to any one of the preceding claims, wherein prior to calculating the behavior of an accommodation signal of the eye, the method comprises performing filtration and averaging of said information acquired about the eye.

**11.** The method according to any one of the preceding claims, wherein the optical powers used refer to the optical powers of a phoropter, of test glasses, or of lenses or systems of variable optical power.

**12.** The method according to claim 1, wherein the information acquired about the eye comprises one or more images of the eye acquired with an optical wavefront sensor.

**13.** A computer program product including code instructions which, when implemented in a computing device, execute a method according to any one of claims 1 to 12.

**Fig. 1**

**Fig. 2**

(A)

(B)

**Fig. 3**

Europäisches
Patentamt

European
Patent Office

Office européen
des brevets

# EUROPEAN SEARCH REPORT

Application Number

EP 20 38 2251

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/174535 A1 (LAI SHUI T [US] ET AL) 11 August 2005 (2005-08-11) * paragraphs [0062], [0087], [0092] * ----- | 1-13 | INV. A61B3/028 A61B3/09 |
| A | US 2008/291395 A1 (DAI GUANG-MING [US] ET AL) 27 November 2008 (2008-11-27) * paragraphs [0053], [0060], [0062], [0063] * ----- | 1-13 | |

TECHNICAL FIELDS
SEARCHED (IPC)

A61B

The present search report has been drawn up for all claims

| Place of search | Date of completion of the search | Examiner |
|---|---|---|
| Munich | 18 September 2020 | Martelli, Luca |

EPO FORM 1503 03.82 (P04C01)

**EP 3 888 525 A1**

## ANNEX TO THE EUROPEAN SEARCH REPORT
## ON EUROPEAN PATENT APPLICATION NO.

EP 20 38 2251

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2020

| Patent document cited in search report | | Publication date | Patent family member(s) | | Publication date |
|---|---|---|---|---|---|
| US 2005174535 | A1 | 11-08-2005 | US 2005174535 A1 | | 11-08-2005 |
| | | | US 2007109498 A1 | | 17-05-2007 |
| | | | US 2009161070 A1 | | 25-06-2009 |
| US 2008291395 | A1 | 27-11-2008 | AU 2008256709 A1 | | 04-12-2008 |
| | | | CA 2688170 A1 | | 04-12-2008 |
| | | | EP 2146621 A1 | | 27-01-2010 |
| | | | US 2008291395 A1 | | 27-11-2008 |
| | | | US 2009073380 A1 | | 19-03-2009 |
| | | | US 2011116039 A1 | | 19-05-2011 |
| | | | US 2012033183 A1 | | 09-02-2012 |
| | | | US 2012271413 A1 | | 25-10-2012 |
| | | | US 2014055750 A1 | | 27-02-2014 |
| | | | US 2015133900 A1 | | 14-05-2015 |
| | | | US 2016135676 A1 | | 19-05-2016 |
| | | | US 2016287066 A1 | | 06-10-2016 |
| | | | WO 2008148038 A1 | | 04-12-2008 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10444539 B2
- EP 2369972 B1
- US 8985768 B2
- US 9462939 B2
- US 2011013140 A

**Non-patent literature cited in the description**

- **W. N. CHARMAN ; G. HERON.** Microfluctuations in accommodation: An update on their characteristics and possible role. *Ophthalmic Physiol. Opt.,* 2015, vol. 35 (5), 476-499
- **W. N. CHARMAN.** The eye in focus: accommodation and presbyopia. *Clin. Exp. Optom.,* 2008, vol. 91 (3), 207-225